**Europäisches Patentamt**

**European Patent Office**

**Office européen des brevets**

(19)

(11) Veröffentlichungsnummer : **0 468 245 B1**

(12) # EUROPÄISCHE PATENTSCHRIFT

(45) Veröffentlichungstag der Patentschrift :
**20.04.94 Patentblatt 94/16**

(51) Int. Cl.$^5$ : **A61K 31/185,** A61K 9/20, A61K 9/28, A61K 9/46, A61K 9/16

(21) Anmeldenummer : **91111125.0**

(22) Anmeldetag : **04.07.91**

(54) **Tablette und Granulat welche als Wirkstoff Mesna enthalten.**

(30) Priorität : **16.07.90 DE 4022525**

(43) Veröffentlichungstag der Anmeldung :
**29.01.92 Patentblatt 92/05**

(45) Bekanntmachung des Hinweises auf die Patenterteilung :
**20.04.94 Patentblatt 94/16**

(84) Benannte Vertragsstaaten :
**AT BE CH DE DK ES FR GB GR IT LI LU NL SE**

(56) Entgegenhaltungen :
**EP-A- 0 198 542**

(73) Patentinhaber : **ASTA Medica Aktiengesellschaft**
**An der Pikardie 10**
**D-01277 Dresden (DE)**

(72) Erfinder : **Sauerbier, Dieter**
**Rauhe Horst 18**
**W-4806 Werter (DE)**
Erfinder : **Engel, Jürgen Prof. Dr.**
**Erlenweg 3**
**W-8755 Alzenau (DE)**
Erfinder : **Milsmann, Eckhard, Dr.**
**Akazienstrasse 23**
**W-4800 Bielefeld 14 (DE)**

Jouve, 18, rue Saint-Denis, 75001 PARIS

**Beschreibung**

Der chemische Name für den Wirkstoff Mesna ist Natrium-2-mercaptoethansulfonat.

Mesna schützt beispielsweise die harnableitenden Organe bei der Therapie von Tumorerkrankungen mit Ifosfamid. Außerdem wird Mesna seit langer Zeit bereits als Mucolytikum eingesetzt.

Mesna ist ein weißes, hygroskopisches Pulver von charakteristischem Geruch. Die Substanz ist stark oxidationsempfindlich und setzt sich bei Kontakt mit Sauerstoff insbesondere in feuchter Athmosphäre schnell zu Dimesna um.

Mesna wird bisher oral, parenteral und inhalativ appliziert. Bei allen verwendeten Darreichungsformen handelt es sich um flüssige Zubereitungen. Für die orale Anwendung wird nur eine wäßrige Lösung angeboten. Da Mesna sehr oxidationsempfindlich ist und in Gegenwart von Sauerstoff zu dem schwer resorbierbaren Dimesna reagiert, muß die wäßrige Lösung vor Sauerstoff geschützt sein. Zu diesem Zweck wird die Lösung in Glasampullen eingeschweißt. Die Ampulle muß vom Patienten vor der Anwendung erst geöffnet werden. Dies ist für die orale Anwendung ein umständliches Vorgehen. Zudem hat die Lösung ein relativ hohes Gewicht und beansprucht viel Platz zur Lagerung.

Der wesentliche Nachteil der Trinklösung ist der sehr unangenehme Geschmack, so daß sie trotz Aromatisierung von den Patienten sehr ungern eingenommen wird. Da Patienten bei der Behandlung mit Zytostatika ohnehin sehr häufig an Übelkeit und Appetitlosigkeit leiden, beeinträchtigt der schlechte Geschmack zusätzlich die Compliance. Aus den oben beschriebenen Gründen besteht seit langer Zeit das Bedürfnis nach einer wohlschmeckenden, stabilen und einzeldosierten oralen Darreichungsform.

Obwohl Mesna als kristallines Pulver vorliegt, scheiterten bisher alle Versuche, Tabletten, überzogene Tabletten oder Weichgelatinekapseln herzustellen. Bei den Weichgelatinekapseln wurde durch Einbettung in eine Lipidmatrix ein guter Schutz vor Sauerstoff und Zersetzung erreicht. Durch Interaktion von Mesna mit der Kapselhülle kam es jedoch immer wieder zum Platzen der Kapseln während der Lagerung: die Kapseln waren physikalisch instabil. Eine Direkttablettierung des Wirkstoffs gelang ebenfalls nicht wegen des hohen Schüttvolumens von bis zu 500 ml/100 g.

Eine wäßrige Granulation des Wirkstoffs zur Vorbereitung der Tablettierung gelang weder durch konventionelles Anteigen noch durch Wirbelschichtgranulation, da sich Mesna bei Kontakt mit Wasser sofort verflüssigt.

Überraschenderweise wurde nun gefunden, daß sich Mesna gut mit Isopropanol oder einer Mischung von Wasser und Isopropanol granulieren läßt und sich das erhaltene Granulat unter Verwendung weiterer Hilfsstoffe gut zu Tabletten und gefilmten Tabletten weiterverarbeiten läßt. Diese Tabletten weisen eine gute chemische Stabilität auf, sind leicht einzunehmen und bei sofortigem Herunterschlucken praktisch geschmacksneutral.

Die Erfindung betrifft somit eine Tablette, die bezogen auf einen Gewichtsteil Mesna

0,01 - 1 Gewichtsteile eines Bindemittels,

0,03 - 0,4 Gewichtsteile eines Sprengmittels,

0,01 - 0,2 Gewichtsteile eines Gleitmittels und

0,1 - 1 Gewichtsteile eines Füllmittels sowie im Falle einer Brausetablette noch zusätzlich 0,05 - 30 Gewichtsteile einer üblichen physiologisch unbedenklichen Brausemischung enthält. Außerdem kann eine solche Tablette gegebenenfalls noch übliche physiologisch unbedenkliche Geschmack-, Süß- und/oder Aromastoffe enthalten.

Weiterhin betrifft die Erfindung ein für eine solche Tablette verwendbares Granulat.

Die Gewichtsmenge an Mesna in einer solchen Tablette beträgt im allgemeinen zwischen 10 und 80 Gewichtsprozent, der Rest sind die zuvor angegebenen Hilfsstoffe, wobei deren Menge jeweils auf einen Gewichtsteil Mesna bezogen ist. Beispielsweise enthalten die erfindungsgemäßen Zubereitungen 100 mg bis 800 mg Mesna, vorzugsweise 300 mg bis 500 mg Mesna.

Die Erfindung betrifft weiterhin ein Verfahren zur Herstellung solcher stabiler Mesna-Tabletten, die als Wirkstoff Mesna (beispielsweis 10 - 80 Gewichts%), gegebenenfalls übliche Geschmack-, Süß- und Aromastoffe sowie jeweils bezogen auf einen Gewichtsteil Mesna

0,01 - 1 Gewichtsteile eines Bindemittels

0,03 - 0,4 Gewichtsteile eines Sprengmittels,

0,01 - 0,2 Gewichtsteile eines Gleitmittels

0,1 - 1 Gewichtsteile eines Füllmittels sowie im Falle einer Brausetablette noch zusätzlich 0,05 - 30 Gewichtsteile einer üblichen physiologisch unbedenklichen Brausemischung enthalten sowie die Herstellung eines entsprechenden Granulats, wobei die Herstellung so erfolgt, daß man Mesna zuerst mit einem $C_1$-$C_4$-Alkohol oder Aceton oder einer Mischung der genannten organischen Mittel mit Wasser und einem Füllmittel sowie Bindemitteln, gegebenenfalls auch einem Sprengmittel in an sich bekannter Weise granuliert, wobei die genannten Hilfsstoffe in den auf einen Gewichtsteil Mesna bezogenen Mengen verwendet werden oder auch nur einen Teil dieser Mengen zusetzt und den Rest nach der Granulierung zufügt, das erhaltene Granulat trocknet und anschließend das Gleitmittel und gegebenenfalls den Rest an Binde-, Füll- und Sprengmittel zusetzt, das ganze homogenisiert, anschließend zu Tabletten verpreßt und gegebenenfalls die so erhaltenen Tabletten in hierfür bekannter Weise mit einem Filmüberzug versieht. Im Falle einer Brausetablette wird nach der Trocknung des Granulats ebenfalls die organische Säure der Brausemischung oder auch die gesamte Brausemischung zugesetzt.

Weitere Ausführungsarten der Erfindung sind in den Patentansprüche angegeben.

Beispiele für die erfindungsgemäßen Hilfs- und Trägerstoffe gemäß den Ansprüchen sind:

Füllmittel: zum Beispiel Stärke, Cellulose, Milchzucker, Saccharose, Fructose, Sorbit, Mannit, Calciumphosphat, Calciumhydrogenphosphat.
Die Gesamtmenge an Füllmittel in der Tablette beträgt 0,1 - 1 Gewichtsteile bezogen auf einen Gewichtsteil Mesna.

Bindemittel: zum Beispiel Gelatine, Celluloseether, Pektine, Alginate (Natriumalginat), Polyvinylpyrrolidon, Lactose, mikrokristalline Cellulose (Avicel).
Die Gesamtmenge an Bindemittel in der Tablette beträgt 0.01 - 1 Gewichtsteile bezogen auf eine Gewichsteil Mesna.

Sprengmittel: zum Beispiel Alginate, Stärke (Maisstärke), Pektine, Carboxymethylcellulose, Ultramylepektin, Bentonite, Polyvinylpolypyrrolidon (bei Polyvinylpolypyrrolidon sind die fadenförmigen Makromoleküle des Polyvinylpyrrolidons quervernetzt; das Produkt ist in allen üblichen Lösemitteln unlöslich; es hat poröse Struktur und ist stark quellfähig;
Kornverteilung: ca. 50 % > 50$\mu$m,
    maximal 1 % > 250$\mu$m;
Schüttdichte: 280 - 380 g/Liter
Die Gesamtmenge an Sprengmittel in der Tablette kann 1 - 10 Gewichts% betragen.

Gleitmittel: zum Beispiel Stearinsäure, Stearate, Polyglykole, Talkum, hochdisperses Siliciumdioxid.
Die Menge Gleitmittel pro 1 Gewichtsteil Mesna beträgt im Falle einer üblichen Schlucktablette vorzugsweise 0,01 - 0,1, insbesondere 0,01 - 0,05 Gewichtsteile. Im Falle einer Brausetablette verwendet man vorzugsweise 0,05-0,15 Gewichtsteile Gleitmittel auf 1 Gewichtsteil Mesna. Die Gesamtmenge an Gleitmittel in der Tablette kann 0,2-10 Gewichts% betragen.

Vorzugsweise werden als Füllmittel Milchzucker (zum Beispiel grobkörnig) und/oder Calciumhydrogenphosphat, als Bindemittel Polyvinylpyrrolidon und/oder mikrokristalline Cellulose (Avicel), als Sprengmittel Stärke, vorzugsweise Maisstärke und als Gleitmittel Stearate (zum Beispiel Magnesiumstearat) verwendet.

Auf 1 Gewichtsteil Mesna kommen zum Beispiel:

Lactose 0,06 - 0,4 Gewichtsteile, vorzugsweise 0,13 - 0,33, insbesondere 0,2 - 0,25 Gewichtsteile. Calciumhydrogenphosphat 0,1 - 0,3 Gewichtsteile, vorzugsweise 0,1 - 0,23, insbesondere 0,1 - 0,12 Gewichtsteile.

Maisstärke (für die Granulatphase) 0,03 - 0,3 Gewichtsteile, vorzugsweise 0,13 - 0,33, insbesondere 0,2 - 0,25 Gewichtsteile.

Polyvinylpyrrolidon 0,02 - 0,1 Gewichtsteile, vorzugsweise 0,03 - 0,06, insbesondere 0,04 - 0,06 Gewichtsteile. Mikrokristalline Cellulose (Avicel) 0,03 - 0,3 Gewichtsteile, vorzugsweise 0,05 - 0,25, insbesondere 0,08 - 0,1 Gewichtsteile.

Maisstärke (für die äußere Phase, das heißt nach Granulierung) 0,02 - 0,25 Gewichtsteile, vorzugsweise 0,03 - 0,1, insbesondere 0,06 - 0,07 Gewichtsteile.

Magnesiumstearat 0,01 - 0,03 Gewichtsteile, vorzugsweise 0,01 - 0,02, insbesondere 0,015 - 0,018 Gewichtsteile.

Das Verhältnis der Hilfsstoffe zueinander liegt zum Beispiel im Bereich

Lactose : Calciumhydrogenphosphat : Maisstärke : Polyvinylpyrrolidon (Granulatphase) : Avicel : Maisstärke : Magnesiumstearat (äußere Phase) wie 1 : 0,4 : 0,4 : 0,2 : 0,4 : 0,3 : 0,07.

Werden andere Bindemittel als Polyvinylpyrrolidon eingesetzt, kommen beispielsweise pro 1 Gewichtsteil Mesna folgende Mengen in Frage:

Gelatine        0,01 - 0,06 Teile pro ein Gewichtsteil Mesna

Celluloseether  0,01 - 0,04 Teile pro ein Gewichtsteil Mesna

Pektin          0,01 - 0,04 Teile pro ein Gewichtsteil Mesna

Natriumalginat  0,01 - 0,01 Teile pro ein Gewichtsteil Mesna

Vorzugsweise enthält das Granulat neben dem Wirkstoff Mesna Milchzucker, Calciumhydrogenphosphat, Maisstärke und Polyvinylpyrrolidon (als Bindemittel).

Calciumhydrogenphosphat dient zum Beispiel als Füllmittel und die Maisstärke dient vorzugsweise als Tablettensprengmittel. In der äußeren Tablettenphase werden vorzugsweise Maisstärke - wieder als Sprengmittel - und mikrokristalline Cellulose (Avicel) als Trockenbindemittel eingesetzt.

Als Milchzucker wird vorzugsweise ein Milchzucker mit folgender Korngrößenfraktion eingesetzt:

Kornverteilung     100 % < 800 µm

12 - 35 % < 400 µm

maximal 7 % < 200 µm

Darüberhinaus ist es günstig, wenn der verwendete Milchzucker noch folgende Bedingungen erfüllt:

Schüttgewicht:             560 g/Liter

Stampfgewicht:            620 g/Liter

Böschungswinkel (Rieselfähigkeit)  35 °

Als Polyvinylpyrrolidon können zum Beispiel die Typen K 25, K 30 und K 90 eingesetzt werden. Polyvinylpyrrolidone werden mit dem K-Wert charakterisiert, der sich aus der relativen Viskosität errechnet. Er ist ein Maß für den Polymerisationsgrad und das Molekulargewicht. Die zuvor erwähnten Polyvinylpyrrolidone sind durch folgende Parameter charakterisiert:

Molekulargewichte:

PVP K 25 =     29 000
PVP K 30 =     45 000
PVP K 90 =     1 100 000

Korngrößenverteilung in %:

| | >50µm > | 100 µm > | 200 µm | >250 µm |
|---|---|---|---|---|
| PVP K 25 | 90 % | 50 % | 5 % | maximal 1 % |
| PVP K 30 | 90 % | 50 % | 5 % | maximal 1 % |
| PVP K 90 | | | 95 % | 90 % |

Schüttdichte:

PVP K 25 =     400 - 600 g/Liter
PVP K 30 =     400 - 600 g/Liter
PVP K 90 =     110 - 250 g/Liter

Zur Granulation des Wirkstoffs Mesna wird vorzugsweise eine Mischung von Isopropanol und Wasser eingesetzt, die zum Beispiel 10 % bis 50 %, vorzugsweise 20 % bis 40 % und insbesondere 30 % bis 35 % Wasser enthält. Bezogen auf den Wirkstoff Mesna werden von dieser Granulierflüssigkeit beispielsweise 0,3 bis 3 Teile, vorzugsweise 0,5 bis 2 Teile und insbesondere 0,7 bis 1 Teil eingesetzt. Anstelle von Isopropanol können auch andere $C_1$-$C_4$-Alkohole (wie zum Beispiel Methanol, Ethanol) oder Aceton verwendet werden. Auch diese Mittel können als Gemisch mit Wasser eingesetzt werden, wobei dieselben Mischverhältnisse wie für die Isopropanol-Wasser-Gemische in Frage kommen. Außerdem ist es vorteilhaft, der Granulierlösung ein Bindemittel wie Polyvinylpyrrolidon zuzusetzen, wobei die Menge an Polyvinylpyrrolidon beispielsweise 0,1 % bis 10 %, vorzugsweise 1 % bis 5 % und insbesondere 3 % bis 5 % beträgt.

Zusätzlich kann es vorteilhaft sein, dem Granulat weitere übliche pharmazeutische Hilfsstoffe und Trägerstoffe zuzusetzen bzw. diese mitzugranulieren.

Ferner können die Tabletten noch enthalten:

Gegenklebemittel, Resorptionsbeschleuniger, Hydrophilisierungsmittel, Feuchthaltemittel und hierzu äquivalente Mittel, sowie übliche Geschmack-, Süß- und Aromastoffe.

Falls es sich bei der erfindungsgemäßen Tablette um eine Brausetablette handelt, enthält diese noch zusätzlich eine übliche physiologisch unbedenkliche Brausemischung. Die Menge dieser Brausemischung bezogen auf einen Gewichtsteil Mesna beträgt 0,05 - 30 Gewichtsteile. Eine solche Brausemischung besteht aus einer Substanz, die in Gegenwart einer Säure in wässrigem beziehungsweise alkoholischem Milieu Kohlendi-

oxid entwickelt ($CO_2$-Lieferer) und einer physiologisch verträglichen organischen Säure.

Als $CO_2$-liefernde Substanzen kommen beispielsweise in Frage: Carbonate oder Hydrogencarbonate des Natriums, Kaliums, Magnesiums oder Calciums, im Falle von Magensium und Calcium vorzugsweise die neutralen Carbonate. Auch Mischungen dieser Carbonate sind möglich. Als organische Säuren, die aus solchen Carbonaten Kohlendioxid freisetzen, kommen beispielsweise in Frage: organische gesättigte oder ungesättigte Di- und Tricarbonsäuren mit 2 bis 8, vorzugsweise 2 bis 6 C-Atomen, die auch eine, zwei, drei oder vier, vorzugsweise eine oder zwei Hydroxygruppen enthalten können. Beispiele hierfür sind: Citronensäure, Weinsäure, Adipinsäure, Maleinsäure, Fumarsäure. Ebenfalls ist Alginsäure verwendbar. Auch Gemische der genannten Säuren sind möglich.

Auf einen Gewichtsteil Mesna werden beispielsweise 0,02 - 18 Gewichtsteile der $CO_2$-liefernden Komponente der Brausemischung und 0,03 - 12 Gewichtsteile der zugehörigen Säurekomponente verwendet. Bei der Herstellung der Brausetablette kann zum Beispiel die komplette Brausemischung als solche nach der Granulierungsstufe zugesetzt werden. Es ist aber auch möglich beziehungsweise bevorzugt, die Komponente der Brausemischung, die das Kohlendioxid liefert, vor der Granulierung zuzusetzen und die entsprechende zugehörige Säurekomponente dann später dem trockenen Granulat zusammen mit beispielsweise dem Gleitmittel, den Geschmack-, Süß- und Aromastoffen sowie gegebenenfalls den restlichen Füll-, Binde- und Sprengmitteln beizumischen.

Zur besseren Abdeckung des unangenehmen Geschmacks hat es sich bewährt, die Mesna Schluck-Tabletten mit einem geeigneten Überzug zu versehen. Dieser Überzug kann eine konventionelle Zuckerdragierung oder aber auch ein polymerhaltiger Filmüberzug sein. Beispielsweise kommen hierfür in Frage: Copolymerisat auf Basis von Dimethylaminoethylmethacrylat und neutralen Methacrylsäureestern (zum Beispiel Eudragit E), Hydroxypropylmethylcellulose, Molekulargewicht 11 000 (zum Beispiel Pharmacoat 606), Ethylcellulose (zum Beispiel Aquacoat).

Die Herstellung der Tabletten (alle Schritte außer der Trocknung) erfolgt zum Beispiel bei Temperaturen zwischen 10 °C und 80 °C vorzugsweise 18 °C bis 50 °C insbesondere 20 °C bis 30 °C.

Die Trocknung des Granulats erfolgt beispielsweise zwischen 40 °C und 80°C vorzugsweise 50 °C bis 70 °C. Falls das Granulat als solches als Arzneimittel verwendet wird, beispielsweise als Trinkgranulat, enthält dieses vorzugsweise neben dem Wirkstoff Mesna nur ein Bindemittel. Bei dem Bindemittel kann es sich um eines der angegebenen Bindemittel handeln, aber es können auch Gemische solcher Bindemittel verwendet werden. Vorzugsweise verwendet man 0,1 - 1 Gewichtsteile Bindemittel auf einen Gewichtsteil Mesna. Die obere Korngröße eines solchen Granulates liegt bei 3 mm.

Das Granulat enthält beispielsweise 100 mg bis maximal 2 g Mesna. Das Mesna ist im allgemeinen in einer solchen Menge in dem Granulat vorhanden, daß es 10 - 80 Gewichtsprozent der Granulatmasse ausmacht. Bindemittel, Füllmittel und gegebenenfalls Gleitmittel bewegen sich in der gleichen Größenordnung wie bei den Tabletten.

Zur Geschmackskaschierung empfiehlt es sich, das Granulat, welches direkt eingenommen wird, mit einem Überzug zu versetzen. Es handelt sich hier um die bekannten und üblichen Überzüge, die sich zum Beispiel erst im sauren Magensaft auflösen oder aber auch magensaftresistent sein können und dann erst im Intestinaltrakt in Lösung gehen.

Es empfiehlt sich darüberhinaus, dem Granulat Geschmack-, Süß- und/oder Aromastoffe zuzumischen.

Als Aromastoffe kommen zum Beispiel folgende Pulveraromen in Frage: Ananas, Apfel, Aprikose, Himbeere, Kirsche, Kola, Orange, Passionsfrucht, Zitrone, Grapefruit.

Das Granulat soll 0,05 - 0,2 Gewichtsteile Aroma, bezogen auf 1 Gewichtsteil Mesna, enthalten.

Als Süßungsmittel können folgende Stoffe eingesetzt werden: Saccharin und sein Natriumsalz, Cyclaminsäure und ihr Natriumsalz, Ammoniumglycyrrhizinat, Fructose, Saccharose, Xylit, Glucose, Sorbit, Mannit. Das Granulat soll 0,003 - 12 Gewichtsteile Süßungsmittel, bezogen auf 1 Gewichtsteil Mesna, enthalten.

Man kann das Granulat zur Geschmacksverbesserung mit einem in Wasser unlöslichen Lack überziehen. Folgende Substanzen werden beispielsweise für diesen Zweck verwendet: Copolymerisate von Dimethylaminomethacrylsäure und neutralen Methacrylsäureestern, die in Wasser und Speichel unlöslich sind, im sauren Bereich sich aber auflösen (wie zum Beispiel Eudragit® E).

Man kann aber auch magensaftresistente Lacke einsetzen, wie zum Beispiel Schellack, Celluloseacetatphthalat, Hydroxypropylmethylcellulosephthalat oder Polymerisat von Methacrylsäure und Methacrylsäureestern (zum Beispiel Eudragit® L und S). Diesen magensaftresistenten Granulaten kann man zur Stabilisierung organische Säure zusetzen, um die applizierbare Suspension schwach sauer einzustellen. Auf das Granulat sollen 0,0125 - 2 Gewichtsteile Lacktrockensubstanz pro 1 Gewichtsteil Mesna aufgetragen werden.

Die angegebenen Geschmack-, Süß- und Aromastoffe kommen in den angegebenen Mengen auch für die erfindungsgemäßen Mesna-Tabletten in Frage. Dasselbe gilt auch für die Überzüge.

Beispiel 1:

Zur Herstellung eines Granulates für 4000 Tabletten mit 300 mg Mesna pro Tablette werden 1200 g Mesna, 280 g Lactose, 120 g Calciumhydrogenphosphat und 120 g Maisstärke durch ein Sieb der Maschenweite 0,8 mm gegeben und anschließend 15 Minuten lang in einem geeigneten Mischer homogenisiert. Anschließend wird mit einer Mischung von 616 g Isopropanol und 284 g Wasser, die 60 g Polyvinylpyrrolidon enthält, angefeuchtet und die feuchte Masse durch ein Sieb der Maschenweite 2 mm passiert. Die Trocknung des Granulates erfolgt beispielsweise in der Wirbelschicht bei einer Zulufttemperatur von 70 °C über einen Zeitraum von 8 Minuten.

Das getrocknete Granulat wird dann beispielsweise zusammen mit 80 g Maisstärke und 120 g mikrokristalliner Cellulose durch ein 0,8 mm-Sieb gegeben und 20 Minuten in einem geeigneten Mischer homogenisiert. Anschließend werden 20 g Magnesiumstearat hinzugefügt und nochmals 2 Minuten gemischt. Die so erhaltene Preßmasse wird auf einer geeigneten Tablettenpresse beispielsweise zu Tabletten mit folgenden tehnologischen Daten verpreßt:

Sollmasse         500,0 mg
Durchmesser       11,0 mm
Wölbungsradius    8,5 mm
Dicke             5,4 - 5,6 mm

Die Tabletten können anschließend in einem geeigneten Gerät mit 225 g einer Filmsuspension (enthaltend eine übliche polymerhaltige Überzugsmasse oder eine übliche zuckerhaltige Überzugsmasse) kontinuierlich besprüht werden, so daß die einzelne Tablette einen Film von 15 mg Gewicht erhält.

Beispiel 2:

Zur Herstellung eines Mesna-Granulates werden 4 kg Mesna Reinsubstanz mit 1,9 kg einer Lösung von 114 g Polyvinylpyrrolidon mit einem K-Wert von 25 in einer Mischung aus 1126 g Isopropanol und 660 g Wasser angefeuchtet und durch ein 2 mm-Sieb granuliert. Diese feuchte Masse wird in der Wirbelschicht bis zu einer Restfeuchte von weniger als 30 % relative Feuchte getrocknet und auf eine maximale Korngröße von 1 mm gesiebt. Zur Geschmackskaschierung wird dieses Granulat in einem Wirbelschichtsprühgerät mit einer Suspension folgender Zusammensetzung überzogen:

```
Eudragit® E (12,5%ige Lösung          4,80 kg
in Isopropanol/Aceton)
Magnesiumstearat                      0,48 kg
Isopropanol                           6,72 kg
                                     12,00 kg
```

Eudragit E ist ein handelsübliches kationisches Polymerisat auf Methacrylatbasis zum Überziehen von Arzneiformen.

Es empfiehlt sich, nach Auftrag von 6 kg Suspension eine Zwischentrocknung durchzuführen und das Granulat durch ein 1,5 mm-Sieb zu geben. Nach Auftrag der gesamten Suspension wird noch einmal auf eine Korngröße von 1,8 mm gesiebt.

Zur Geschmacksverbesserung wird diesem Wirkstoffgranulat ein aromatisiertes Zuckergranulat folgender Zusammensetzung zugesetzt:

Staubzucker       14,400 kg
Aroma (pulvis)    0,406 kg

werden gemischt und mit 0,9 kg Wasser angefeuchtet, durch ein 2 mm-Sieb granuliert und in einem geeigneten Trockner bis zu einer Restfeuchte von weniger als 30 % relative Feuchte getrocknet.

Schließlich werden beide Granulate homogen gemischt und zu 5 g in Beutel dosiert. Ein Beutel enthält 1 g Mesna.

Zur Einnahme wird der Inhalt eines Beutels in 100 mg Wasser suspendiert.

**Patentansprüche**

1. Tablette enthaltend als Wirkstoff Mesna, gegebenenfalls übliche Geschmack-, Süß- und Aromastoffe, sowie jeweils bezogen auf einen Gewichtsteil Mesna
   0,01 - 1 Gewichtsteile eines Bindemittels
   0,03 - 0,4 Gewichtsteile eines Sprengmittels
   0,01 - 0,2 Gewichtsteile eines Gleitmittels
   0,1 - 1 Gewichtsteile eines Füllmittels sowie im Falle einer Brausetablette noch zusätzlich
   0,05-30 Gewichtsteile einer üblichen physiologisch unbedenklichen Brausemischung.

2. Tablette nach Anspruch 1, dadurch gekennzeichnet, daß die Tablette 10 - 80 Gewichtsprozent Mesna enthält, sowie die in Anspruch 1 angegebenen Stoffe, jeweils bezogen auf einen Gewichtsteil Mesna.

3. Tablette nach einem oder mehreren der vorangegangenen Ansprüche, dadurch gekennzeichnet, daß die Tablette mit einem üblichen Film überzogen ist.

4. Verfahren zur Herstellung einer stabilen Tablette enthaltend als Wirkstoff Mesna, gegebenenfalls übliche Geschmack-, Süß- und Aromastoffe, sowie jeweils bezogen auf einen Gewichtsteil Mesna
   0,01 - 1 Gewichtsteile eines Bindemittels
   0,03 - 0,4 Gewichtsteile eines Sprengmittels
   0,01 - 0,2 Gewichtsteile eines Gleitmittels
   0,1 - 1 Gewichtsteile eines Füllmittels sowie im Falle einer Brausetablette noch zusätzlich
   0,05-30 Gewichtsteile einer üblichen physiologisch unbedenklichen Brausemischung
   dadurch gekennzeichnet,
   daß man Mesna zuerst mit einem $C_1$-$C_4$-Alkohol oder Aceton oder einer Mischung der genannten organischen Mittel mit Wasser und einem Füllmittel sowie Bindemitteln, gegebenenfalls auch einem Sprengmittel sowie gegebenenfalls im Falle einer Brausetablette mit dem $CO_2$-Lieferer der Brausemischung in an sich bekannter Weise granuliert, wobei die genannten Hilfsstoffe in den auf einen Gewichtsteil Mesna bezogenen Mengen verwendet werden oder auch nur ein Teil dieser Mengen zusetzt und den Rest nach der Granulierung zufügt, das erhaltene Granulat trocknet und anschließend das Gleitmittel sowie im Falle einer Brausetablette die organische Säure der Brausemischung oder auch die gesamte Brausemischung und gegebenenfalls den Rest an Binde-, Füll- und Sprengmittel sowie gegebenenfalls übliche Geschmack-, Süß- und Aromastoffe zusetzt, das ganze homogenisiert, anschließend zu Tabletten verpreßt und gegebenenfalls die so erhaltenen Tabletten in hierfür bekannter Weise mit einem Filmüberzug versieht.

5. Granulat enthaltend als Wirkstoff Mesna, gegebenenfalls übliche Geschmack-, Süß- und Aromastoffe, sowie jeweils bezogen auf einen Gewichtsteil Mesna
   0,01 - 1 Gewichtsteile eines Bindemittels sowie gegebenenfalls
   0,01 - 0,2 Gewichtsteile eines Gleitmittels
   0,1 - 1 Gewichtsteile eines Füllmittels.

6. Granulat nach einem oder mehreren der vorangegangenen Ansprüche, dadurch gekennzeichnet, daß das Granulat mit einem üblichen Film überzogen ist.

7. Verfahren zur Herstellung eines Mesna-Granulats enthaltend als Wirkstoff Mesna, gegebenenfalls übliche Geschmack-, Süß- und Aromastoffe, sowie jeweils bezogen auf einen Gewichtsteil Mesna
   0,01 - 1 Gewichtsteile eines Bindemittels sowie gegebenenfalls
   0,01 - 0,2 Gewichtsteile eines Gleitmittels
   0,1 - 1 Gewichtsteile eines Füllmittels
   dadurch gekennzeichnet, daß man einen Gewichtsteil Mesna mit einem $C_1$-$C_4$-Alkohol oder Aceton oder einer Mischung der genannten organischen Mittel mit Wasser und
   0,01 - 1 Gewichtsteilen eines Bindemittels sowie gegebenenfalls
   0,1 - 1 Gewichtsteilen eines Füllmittels und/oder
   0,01 - 0,2 Gewichtsteilen eines Gleitmittels
   granuliert, das erhaltene Granulat trocknet, das trockene Granulat gegebenenfalls mit einem Filmüberzug versieht und gegebenenfalls übliche Geschmack-, Süß- und Aromastoffe zusetzt, das ganze homogenisiert und gegebenenfalls das so erhaltene Produkt in hierfür bekannter Weise mit einem Filmüberzug versieht.

**Claims**

1. A tablet containing mesna as active substance, optional conventional flavouring, sweetening and aromatic substances, as well as
0.01 - 1 parts by weight of a binder
0.03 - 0.4 parts by weight of a disintegrating agent
0.01 - 0.2 parts by weight of a lubricant
0.1 - 1 parts by weight of a filler as well as, in the case of an effervescent tablet, an additional
0.05 - 30 parts by weight of a conventional physiologically acceptable effervescent mixture, each being with respect to one part by weight of mesna.

2. A tablet according to Claim 1, characterised in that the tablet contains 10 - 80 parts by weight of mesna and the substances cited in Claim 1, each being with respect to one part by weight of mesna.

3. A tablet according to one or more of the preceding Claims, characterised in that the tablet is coated with a conventional film.

4. A process for the preparation of a stable tablet containing mesna as active substance, optional conventional flavouring, sweetening and aromatic substances, as well as
0.01 - 1 parts by weight of a binder
0.03 - 0.4 parts by weight of a disintegrating agent
0.01 - 0.2 parts by weight of a lubricant
0.1 - 1 parts by weight of a filler as well as, in the case of an effervescent tablet, an additional
0.05 - 30 parts by weight of a conventional physiologically acceptable effervescent mixture, each being with respect to one part by weight of mesna, characterised in that mesna is first granulated in a way which is known per se with a $C_1$-$C_4$-alcohol or acetone or a mixture of the organic agents mentioned with water and a filler and binders, optionally also a disintegrating agent and optionally, in the case of an effervescent tablet, with the $CO_2$ supplier from the effervescent mixture, wherein the auxiliary substances mentioned are used in the amounts relating to one part by weight of mesna or else only a part of these amounts is used and the remainder is added after granulation, the granulate obtained is dried and then the lubricant and, in the case of an effervescent tablet, the organic acid from the effervescent mixture or else the whole effervescent mixture and optionally the remainder of the binder, filler and disintegrating agent as well as optional flavouring, sweetening and aromatic substances, are added, the entire mixture is homogenised and then compressed to form tablets and optionally the tablets obtained in this way are provided with a film coating in a known manner.

5. A granulate containing mesna as active substance, optional conventional flavouring, sweetening and aromatic substances as well as
0.01 - 1 parts by weight of a binder and optionally
0.01 - 0.2 parts by weight of a lubricant
0.1 - 1 part by weight of a filler,
each being with respect to one part by weight of mesna.

6. A granulate according to one or more of the preceding Claims, characterised in that the granulate is coated with a conventional film.

7. A process for preparing a mesna granulate containing mesna as active substance, optional conventional flavouring, sweetening and aromatic substances, as well as
0.01 - 1 parts by weight of a binder and optionally
0.01 - 0.2 parts by weight of a lubricant
0.1 - 1 part by weight of a filler,
each being with respect to one part by weight of mesna, characterised in that one part by weight of mesna is granulated with a $C_1$-$C_4$-alcohol or acetone or a mixture of the organic agents mentioned with water and
0.01 - 1 parts by weight of a binder and optionally
0.1 - 1 parts by weight of a filler and/or
0.01 - 0.2 parts by weight of a lubricant, the granulate obtained is dried, the dry granulate is optionally provided with a film coating and optionally conventional flavouring, sweetening and aromatic substances are added, the whole mixture is homogenised and the product obtained in this way is optionally provided with a film coating in a known manner.

## Revendications

1. Comprimé contenant comme principe actif du mesna, éventuellement des agents de sapidité, édulcorants et aromatisants usuels, et aussi, toujours par rapport à 1 partie en poids de mesna
   de 0,01 à 1 partie en poids d'un liant,
   de 0,03 à 0,4 partie en poids d'un désintégrant,
   de 0,01 à 0,2 partie en poids d'un lubrifiant,
   de 0,1 à 1 partie en poids d'une charge, et éventuellement, dans le cas d'un comprimé effervescent, de plus
   de 0,05 à 30 parties en poids d'un mélange effervescent usuel physiologiquement inoffensif.

2. Comprimé selon la revendication 1, caractérisé en ce que le comprimé contient de 10 à 80 % en poids de mesna, ainsi que les substances indiquées dans la revendication 1, toujours par rapport à 1 partie en poids de mesna.

3. Comprimé selon l'une ou plusieurs des revendications précédentes, caractérisé en ce que le comprimé est enrobé d'un film usuel.

4. Procédé pour préparer un comprimé stable contenant comme principe actif du mesna, éventuellement des agents de sapidité, édulcorants et aromatisants usuels, et aussi, toujours par rapport à 1 partie en poids de mesna
   de 0,01 à 1 partie en poids d'un liant,
   de 0,03 à 0,4 partie en poids d'un désintégrant,
   de 0,01 à 0,2 partie en poids d'un lubrifiant,
   de 0,1 à 1 partie en poids d'une charge, et éventuellement, dans le cas d'un comprimé effervescent, de plus
   de 0,05 à 30 parties en poids d'un mélange effervescent usuel physiologiquement inoffensif,
   caractérisé en ce qu'on granule d'une manière connue en soi du mesna, tout d'abord avec un alcool en $C_1$-$C_4$ ou de l'acétone ou un mélange des composés organiques mentionnés avec de l'eau, et avec une charge et des liants, éventuellement aussi avec un désintégrant et éventuellement, dans le cas d'un comprimé effervescent, avec le précurseur de $CO_2$ du mélange effervescent, auquel cas les adjuvants mentionnés sont utilisés en les quantités rapportées à 1 partie en poids de mesna, ou encore on n'ajoute qu'une partie de ces quantités, le reste étant ajouté après la granulation, on sèche le granulé obtenu, puis on ajoute le lubrifiant et, dans le cas d'un comprimé effervescent, l'acide organique du mélange effervescent ou aussi la totalité du mélange effervescent et éventuellement le reste de liant, de charge et de désintégrant, et éventuellement des agents de sapidité, édulcorants et aromatisants usuels, on homogénéise l'ensemble, puis on le comprime pour obtenir des comprimés, puis, d'une manière connue, on applique éventuellement sur les comprimés obtenus un film d'enrobage.

5. Granulé contenant comme principe actif du mesna, éventuellement des agents de sapidité, édulcorants et aromatisants usuels, et aussi, toujours pour 1 partie en poids de mesna
   de 0,01 à 1 partie en poids d'un liant et éventuellement
   de 0,01 à 0,2 partie en poids d'un lubrifiant,
   de 0,1 à 1 partie en poids d'une charge.

6. Granulé selon l'une ou plusieurs des revendications précédentes, caractérisé en ce que le granulé est enrobé d'un film usuel.

7. Procédé pour préparer un granulé de mesna, éventuellement des agents de sapidité, édulcorants et aromatisants usuels, et aussi, toujours pour 1 partie en poids de mesna
   de 0,01 à 1 partie en poids d'un liant et éventuellement
   de 0,01 à 0,2 partie en poids d'un lubrifiant,
   de 0,1 à 1 partie en poids d'une charge,
   caractérisé en ce qu'on granule 1 partie en poids de mesna avec un alcool en $C_1$-$C_4$ ou de l'acétone ou un mélange des substances organiques mentionnées, avec de l'eau et avec
   de 0,01 à 1 partie en poids d'un liant et éventuellement

de 0,1 à 1 partie en poids d'une charge et/ou
de 0,01 à 0,2 partie en poids d'un lubrifiant,
on sèche le granulé obtenu, on applique éventuellement sur le granulé séché un film d'enrobage, et éventuellement on ajoute des agents de sapidité, édulcorants et aromatisants usuels, on homogénéise l'ensemble et éventuellement on applique sur le produit ainsi obtenu, d'une manière connue en soi, un film d'enrobage.